(19) **Europäisches Patentamt / European Patent Office / Office européen des brevets**

(11) **EP 4 699 531 A1**

(12) **EUROPEAN PATENT APPLICATION**
published in accordance with Art. 153(4) EPC

(43) Date of publication:
**25.02.2026 Bulletin 2026/09**

(21) Application number: **24792701.5**

(22) Date of filing: **17.04.2024**

(51) International Patent Classification (IPC):
**A61B 5/11** *(2006.01)*        **A61B 5/107** *(2006.01)*

(52) Cooperative Patent Classification (CPC):
**A61B 5/107; A61B 5/11**

(86) International application number:
**PCT/JP2024/015298**

(87) International publication number:
**WO 2024/219429 (24.10.2024 Gazette 2024/43)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **18.04.2023 JP 2023067744**

(71) Applicant: **Orgo Inc.
Sapporo-shi, Hokkaido 060-0042 (JP)**

(72) Inventors:
• **TSUYUKI, Yasuaki
Sapporo-shi, Hokkaido 060-0042 (JP)**

• **UENO, Ryo
Sapporo-shi, Hokkaido 060-0042 (JP)**
• **NUMATA, Ryotaro
Sapporo-shi, Hokkaido 060-0042 (JP)**
• **SAKAGUCHI, Hiroki
Sapporo-shi, Hokkaido 060-0042 (JP)**
• **KOMODA, Noriyoshi
Sapporo-shi, Hokkaido 060-0042 (JP)**

(74) Representative: **SSM Sandmair
Patentanwälte Rechtsanwalt
Partnerschaft mbB
Joseph-Wild-Straße 20
81829 München (DE)**

(54) **EXERCISE EVALUATION DEVICE, EXERCISE EVALUATION METHOD, AND EXERCISE EVALUATION PROGRAM**

(57)     Provided are a motion evaluation device (11), a motion evaluation method, and a motion evaluation program for more accurately grasping a physical condition of an evaluation subject.

The motion evaluation device (11) includes a reference storage unit (23), a posture estimation unit (15), an inside-outside body information calculation unit (19), an evaluation unit (25), and a display control unit (27). The reference storage unit (23) stores reference information as a reference for evaluation. The posture estimation unit (15) estimates a joint position of each of a plurality of joints of an evaluation subject on the basis of moving image data of the evaluation subject evaluated for a motion of a body. The inside-outside body information calculation unit (19) obtains, as inside-outside body information, at least one of a muscle activity, a joint load, a center of pressure, or a reaction force for the evaluation subject by using a height and weight of the evaluation subject and model data. The evaluation unit (25) evaluates the motion of the body of the evaluation subject on the basis of the reference information, a plurality of the joint positions, and the inside-outside body information. The display control unit (27) generates an image indicating an evaluation result.

FIG. 3A

## Description

Technical Field

The present invention relates to a motion evaluation device, a motion evaluation method, and a motion evaluation program.

Background Art

For example, in order to grasp a physical condition of a person having a physical disorder or the like, evaluation (diagnosis or the like) is performed by visual observation or palpation, and these evaluation results are recorded in a medical chart. However, such evaluation is based on subjectivity of a person who performs the evaluation, and evaluation results recorded for an evaluated evaluation subject are inferior in objectivity. Therefore, evaluation having objectivity is required.

Accordingly, Patent Literature 1 proposes a motion evaluation system that evaluates a motion of a body, and the motion evaluation system includes a reference value storage unit, a site specifying unit, and an evaluation unit. The reference value storage unit stores a reference value related to the position of at least one part of the body. The site specifying unit specifies a site of the body by analyzing an image obtained by imaging the body. The evaluation unit compares the position of the part in the image with the reference value to determine an evaluation value of the motion.

Citation List

Patent Literature

Patent Literature 1: JP 2022-43264 A

Summary of Invention

Technical Problem

However, the motion evaluation system described in Patent Literature 1 has a limit in grasping the physical condition.

Therefore, an object of the present invention is to provide a motion evaluation device, a motion evaluation method, and a motion evaluation program for more accurately grasping a physical condition of an evaluation subject.

Solution to Problem

A motion evaluation device of the present invention includes a reference storage unit, a posture estimation unit, an inside-outside body information calculation unit, an evaluation unit, and a display control unit. The reference storage unit stores reference information as a reference for evaluation. The posture estimation unit estimates a joint position of each of a plurality of joints of an evaluation subject on the basis of moving image data of the evaluation subject evaluated for a motion of a body. The inside-outside body information calculation unit obtains, as inside-outside body information, at least one of a muscle activity, a joint load, a center of pressure, or a reaction force for the evaluation subject by using a height and weight of the evaluation subject and model data. The evaluation unit evaluates the motion of the body of the evaluation subject on the basis of the reference information, a plurality of the joint positions, and the inside-outside body information. The display control unit generates an image indicating an evaluation result obtained by the evaluation unit.

It is preferable that the inside-outside body information calculation unit obtains evaluation subject model data adapted to the evaluation subject by performing scaling conversion of the model data in which weight assignment and a gravity center position of a segment at a definite height and weight are set on the basis of the height and weight of the evaluation subject, and obtains the inside-outside body information using the obtained evaluation subject model data.

The moving image data may be data of a moving image captured from a fixed point with the evaluation subject as a subject.

It is preferable that the evaluation unit resamples the inside-outside body information from a first keyframe image to a second keyframe image selected from time-series data constituting the moving image data, and evaluates the motion of the body of the evaluation subject by comparing the inside-outside body information with the reference information.

A motion evaluation method of the present invention includes a reference storage step, a posture estimation step, an inside-outside body information calculation step, an evaluation step, and a display control step. The reference storage step stores reference information as a reference for evaluation. The posture estimation step estimates a joint position of each of a plurality of joints of an evaluation subject on the basis of moving image data of the evaluation subject evaluated for a motion of a body. The inside-outside body information calculation step obtains, as inside-outside body information, at least one of a muscle activity, a joint load, a center of pressure, or a reaction force for the evaluation subject by using a height and weight of the evaluation subject and model data. The evaluation step evaluates the motion of the body of the evaluation subject on the basis of the reference information, a plurality of the joint positions, and the inside-outside body information. The display control unit generates an image indicating an evaluation result obtained in the evaluation step.

A motion evaluation program of the present invention causes a computer to execute each of the above steps.

Advantageous Effects of Invention

According to the present invention, physical information of an evaluation subject is more accurately grasped.

Brief Description of Drawings

Fig. 1 is an explanatory diagram of a motion evaluation system including a motion evaluation device as an embodiment of the present invention.
Fig. 2 is an explanatory diagram of an imaging method performed by a camera.
Fig. 3A is a configuration diagram of a motion evaluation device as an embodiment of the present invention.
Fig. 3B is a configuration diagram of a motion evaluation device as another embodiment.
Fig. 4 is an explanatory diagram illustrating an example of an image displayed on a terminal device.
Fig. 5 is a configuration diagram of a motion evaluation device as another embodiment.

Description of Embodiments

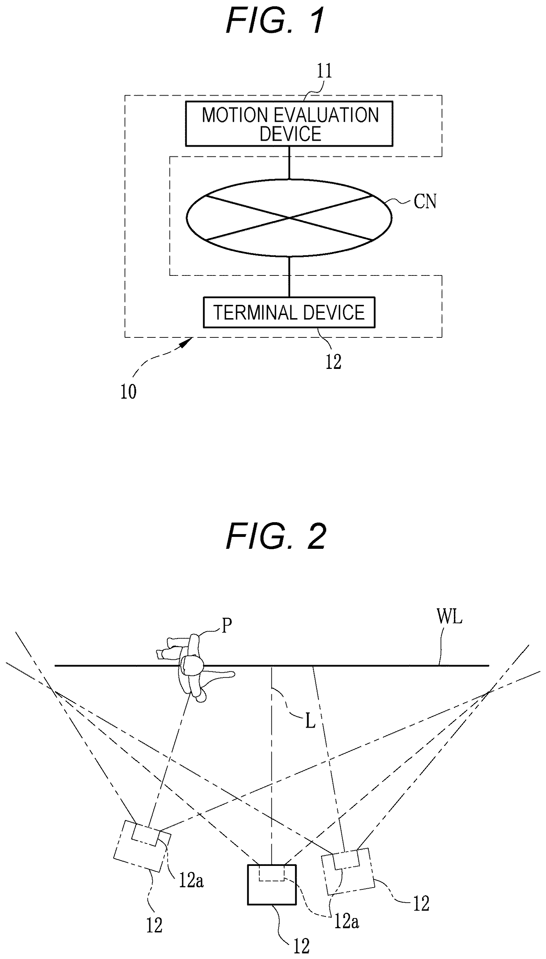

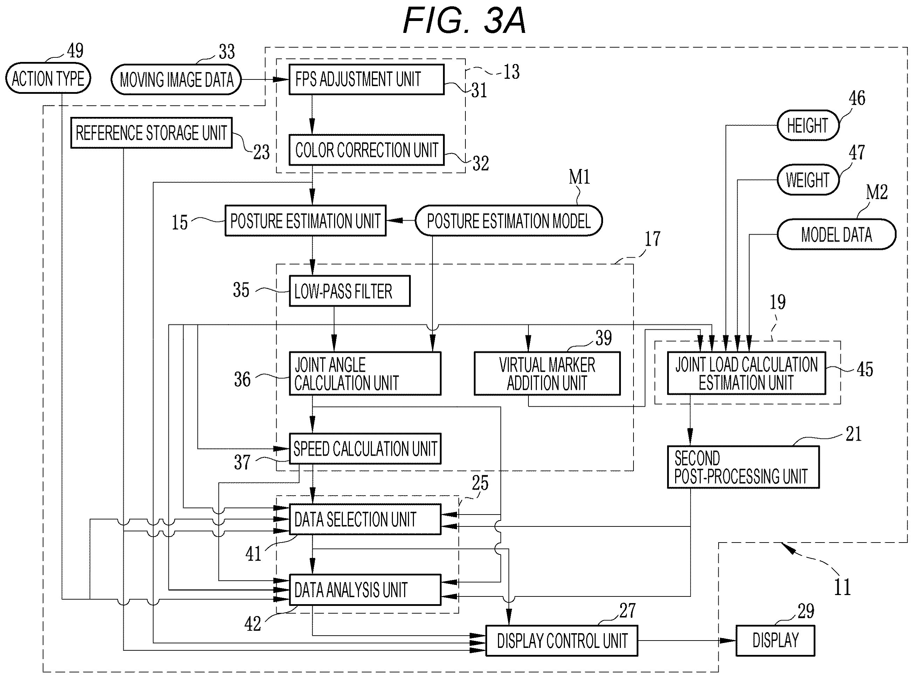

In Fig. 1, a motion evaluation system 10 is for evaluating a motion of a body of an evaluation subject who is a subject of evaluation. The motion evaluation system 10 includes a motion evaluation device 11 and a terminal device 12, which can transmit and receive various data via a communication network CN, and this is also the case in the present example. The terminal device 12 is, for example, a terminal device used by the evaluation subject, and may be any device as long as it includes a camera 12a (see Fig. 2) that captures an image of the evaluation subject, and for example, a smartphone to be described later or the like can be used. The motion evaluation system 10 evaluates the motion of the body of the evaluation subject who is a subject of evaluation by the motion evaluation device 11 on the basis of a moving image captured by the terminal device 12, and displays an evaluation result on a display 29 (see Fig. 3A) which is a display unit of the terminal device 12.

The motion evaluation system 10 may further include a client terminal (not illustrated). The client terminal can transmit and receive various data to and from the motion evaluation device 11 via the communication network CN, and thus the evaluation result is displayed on a display as a display unit of the client terminal. The client terminal is, for example, a terminal device used by a client such as a trainer who instructs on body training of the evaluation subject, a physical therapist, or a therapist, and is not particularly limited as long as it is a computer including a display unit such as a smartphone or a mobile terminal. The terminal device 12 and the client terminal may operate on a browser by transmitting a program that operates on the browser from the motion evaluation device 11, or may operate by incorporating predetermined application software and executing a program of the application software.

The terminal device 12 is provided in a state where the position and orientation are fixed. Thus, the moving image captured by the camera provided in the terminal device 12 is obtained as a fixed point captured moving image captured from a fixed point. It is preferable that the terminal device 12 be arranged so that the entire body of a moving evaluation subject is imaged during an evaluation target time, that is, from the start time to the end time to be evaluated, and this is also the case in the present example. In this example, as illustrated in Fig. 2, a walking line (walking path) WL linearly extending on the floor surface is set as a region where the evaluation subject P moves, and walking of the evaluation subject P from one end to the other end on the walking line WL is an example of motion of the evaluation subject P. When viewed from above, the terminal device 12 is disposed so that an imaging optical axis L of the camera 12a intersects the walking line WL. The height of the camera 12a from the floor surface is set within a range of approximately 0.6 m or more and 1.2 m or less from the floor surface so that the entire body of the evaluation subject P is imaged, and is set to 1 m, for example. In this manner, the entire body from the foot portion to the head is imaged in the evaluation target time of the evaluation subject P walking on the walking line WL. In this example, the terminal device 12 is positioned so that the walking line WL and the imaging optical axis L are orthogonal to each other at the center of the walking line WL. However, when the entire body of the evaluation subject P is imaged, the imaging optical axis L may be positioned to intersect at an angle other than 90° at a position shifted from the center of the walking line WL as in the terminal device 12 indicated by a two-dot chain line in Fig. 2.

The motion evaluation device 11 is for evaluating the motion of the body of the evaluation subject. The motion evaluation device 11 is an example of an embodiment of the present invention, and each unit illustrated in Fig. 3A as an example can be appropriately omitted or replaced with another unit.

The motion evaluation device 11 includes a pre-processing unit 13, a posture estimation unit 15, a first post-processing unit 17, an inside-outside body information calculation unit 19, a second post-processing unit 21, a reference storage unit 23, an evaluation unit 25, and a display control unit 27. The motion evaluation device 11 constitutes the motion evaluation system 10 (see Fig. 1) together with the display 29 of the terminal device 12, but the display 29 may be incorporated as a part of the motion evaluation device 11.

The pre-processing unit 13 includes a frame rate (hereinafter referred to as FPS, and FPS is an abbreviation of frames per second) adjustment unit 31 and a color correction unit 32. The pre-processing unit 13 preferably further includes an acquisition unit (not illustrated) and a calibration processing unit (not illustrated), and is also provided in the present example.

Moving image data 33 input to the pre-processing unit 13 may be either data of a moving image obtained by imaging the evaluation subject as a subject or moving image data generated from a plurality of still images obtained by imaging the evaluation subject as a subject. Examples of the data of the moving image obtained by capturing the evaluation subject as the subject include data captured by a monocular camera provided in a smartphone or the like, and also in the present example, the monocular camera provided in the smartphone as the terminal device 12 (see Figs. 1 and 2) is the camera 12a (see Fig. 2), and the moving image thus captured is used as the moving image data 33.

Since the camera 12a is fixed and imaged as described above, distortion of a subject image changes depending on the position of the subject in the imaging range. The manner of changing distortion varies depending on performance of the lens of the camera 12a and also varies depending on the posture (height and orientation) of the camera 12a. Accordingly, it is preferable that the camera 12a capture a moving image for correcting the distortion and send the captured moving image to the above-described acquisition unit to calibrate internal parameters of the camera 12a before starting the imaging of the evaluation subject P (see Fig. 2). Examples of the moving image for performing calibration include a moving image obtained by imaging a checkerboard while moving in the entire imaging range with a checkerboard in which rectangular regions of white and black are alternately formed as a subject.

The resolution and the FPS in the case of imaging by the camera 12a are not particularly limited, but it is preferable that imaging for calibration and imaging of the evaluation subject P are the same imaging method including the resolution and the FPS so that the internal parameters do not change due to a difference in the imaging method by the camera 12a in terms of using the same internal parameters. In the present example, the imaging for calibration and the imaging of the evaluation subject P are set to 4 K (2160p) having the same resolution and 120 fps having the same FPS. In the imaging of the evaluation subject P, the resolution may be set according to the distance between the evaluation subject P and the camera 12a, and for example, in a case where the distance is as large as 10 m, it is preferable to set the resolution to at least 4 K. In the imaging of the evaluation subject P, the FPS may be set to be high in order to suppress the occurrence of blur, and also in the present example, 240 fps may be set instead of 120 fps. The acquisition unit sends the acquired moving image data 33 to the above-described calibration processing unit in a case where the moving image data for calibration is acquired, and sends the acquired moving image data 33 to the FPS adjustment unit 31 in a case where the moving image data 33 in which the evaluation subject P is imaged is acquired.

In a case where the next imaging is performed with the same imaging method such as the camera 12a to be used, the posture of the camera 12a, and settings of the camera 12a (for example, resolution and angle of view), it is not necessary to perform calibration in the next imaging. This is because the internal parameters do not change. Note that, in the present example, while the above-described terminal device 12 logs in and is connected to the motion evaluation device 11, a button for a calibration execution instruction for instructing to perform calibration is displayed on a touch panel display provided as a display of the terminal device 12. It is configured so that acquisition of a moving image obtained by imaging a checkerboard and execution of calibration by a calibration processing unit are performed by a touch operation on the button.

The calibration processing unit obtains internal parameters of the camera as the camera 12a on the basis of the transmitted moving image data 33, and transmits the obtained internal parameters to the posture estimation unit 15 via the FPS adjustment unit 31 and the color correction unit 32. In a case where the pre-processing unit 13 performs processing of reducing the resolution as described later, the calibration processing unit obtains the internal parameters of the camera 12a after converting the resolution of the sent moving image to be the same as the resolution of a frame image sent from the pre-processing unit 13 as described later. Thus, it is possible to prevent the internal parameters from changing and to maintain estimation accuracy in the posture estimation unit 15.

Depending on the resolution in the acquired moving image data 33, the pre-processing unit 13 preferably further performs resolution reduction processing for reducing the resolution of each frame image from the viewpoint of improving processing efficiency in the next and subsequent steps. The pre-processing unit 13 of the present example reduces the resolution to full HD (high definition, 1080p), and confirms that accuracy in subsequent steps, for example, accuracy in calculation of joint positions described later, and the like are maintained even in the full HD resolution. For example, with respect to the joint positions, it is confirmed that the error is 26.00 cm in the global space and in a case of non-fixation in which the position of the pelvis is not fixed. Further, it has been confirmed that the error in a case where the position of the pelvis is fixed is 12.02 cm. Note that, in a case where the resolution is relatively insufficient, such as a case where the subject image of the evaluation subject P (see Fig. 2) is excessively small in the moving image, it may be preferable not to perform the resolution reduction processing, and thus the resolution reduction processing is performed as necessary. In this manner, the presence or absence of the resolution reduction processing may be determined according to the balance between the size of the subject image of the evaluation subject P in the moving image and the set resolution. Accordingly, in the present example, the resolution reduction processing is not performed in a case where the distance between the evaluation subject P and the camera 12a exceeds about 10 m.

In a case where the moving image data 33 of the evaluation subject is acquired, the FPS adjustment unit 31 performs FPS adjustment processing of reducing the number of frames (the number of images) per second in response to the acquisition, thereby reducing the processing time in the subsequent step. As the subsequent step, for example, there is a step of estimation processing or calculation processing in the inside-outside body information calculation unit 19. The FPS value and the number of frame images due to the decrease in FPS are not particularly limited. In this example, the FPS is reduced to 30 fps, and the FPS of the moving image learned in advance by artificial intelligence (AI) used in the posture estimation unit 15 is also set to 30 fps. In this manner, the FPS of the moving image is reduced by the FPS adjustment unit 31 so as to be the same as the FPS of the moving image learned by the AI used for the posture estimation unit 15, whereby the certainty of estimation in the posture estimation unit 15 is maintained. The FPS adjusted moving image data obtained by the FPS adjustment unit 31 is output to the color correction unit 32.

In a case of performing the resolution reduction processing, the pre-processing unit 13 may first perform either the resolution reduction processing or the FPS reduction processing. The pre-processing unit 13 outputs the time-series data of the frame images obtained through the resolution reduction processing and the FPS reduction processing to the color correction unit 32.

The color correction unit 32 performs color correction processing such as brightness, contrast, and white balance on the FPS adjusted moving image data input from the FPS adjustment unit 31. Thus, for example, artificial intelligence (AI) used for the inside-outside body information calculation unit 19 more reliably recognizes the subject image as a person when it is too dark, for example, depending on the imaging situation. Color-corrected moving image data obtained by the color correction unit 32 is output to the posture estimation unit 15 and the display control unit 27.

A posture estimation model M1 and the AI are incorporated in the posture estimation unit 15, and each of positions of joints (hereinafter referred to as joint positions) of the evaluation subject is estimated from the color-corrected moving image data input from the color correction unit 32 and output in three-dimensional coordinates. The coordinate system is a world coordinate system. The estimation processing is performed by a preset posture estimation model, the posture estimation model M1 has a graph structure having vertices and sides, a joint and an ID (identification information) are associated with each other, and the ID is referred to as a joint ID in the following description. The time-series data of the joint positions obtained by the posture estimation unit 15 is output to a low-pass filter 35 of the first post-processing unit 17.

The first post-processing unit 17 includes a low-pass filter 35, a joint angle calculation unit 36, a speed calculation unit 37, and a virtual marker addition unit 39. The low-pass filter 35 performs frequency filtering processing of removing high frequency components to obtain only low frequency components on the time-series data of the joint positions, and obtains time-series data in which a temporal displacement of the joint positions is smooth. The time-series data (hereinafter referred to as joint position filter data) of the joint position with smooth displacement by the low-pass filter 35 is output to each of the joint angle calculation unit 36, the speed calculation unit 37, a data selection unit 41 and a data analysis unit 42 of the evaluation unit 25, the virtual marker addition unit 39, and the inside-outside body information calculation unit 19. Note that the low-pass filter 35 may be replaced with either a missing value complementing unit that complements a missing value or an abnormal value correction unit that corrects an abnormal value.

In a case where the joint position filter data is input, the joint angle calculation unit 36 calculates an angle between a vertex and a neighboring vertex connected by a side of the posture estimation model as a joint angle. The joint angle calculation unit 36 is connected to the speed calculation unit 37 and the data selection unit 41 and the data analysis unit 42 to be described later of the evaluation unit 25, and outputs the calculated joint angle to the speed calculation unit 37 and the data selection unit 41 and the data analysis unit 42 of the evaluation unit 25.

The speed calculation unit 37 calculates and obtains at least one of a joint position speed or a joint angular speed. The speed calculation unit 37 calculates a speed at which a joint position is displaced as the joint position speed on the basis of the joint position filter data input from the low-pass filter 35. The speed calculation unit 37 calculates the joint angular speed on the basis of the joint angle input from the joint angle calculation unit 36. That is, the joint angular speed is calculated by first differentiating the time-series data of the joint angle.

The virtual marker addition unit 39 calculates a virtual marker on the basis of the joint position. Specifically, the virtual marker addition unit 39 generates a virtual marker that maintains a definite relative distance from determined positions of two or more joint IDs, that is, joint positions, and generates a plurality of virtual markers using a combination of a plurality of joint positions.

The inside-outside body information calculation unit 19 includes a joint load calculation estimation unit 45, a muscle activity estimation calculation unit (not illustrated), a reaction force estimation calculation unit (not illustrated), a COP (abbreviation of center of pressure, sometimes referred to as atsuryoku chushin, or sokuatsu chushin) estimation calculation unit (not illustrated), and a control unit (not illustrated). The control unit integrally controls the joint load calculation estimation unit 45, the muscle activity estimation calculation unit, the reaction force estimation calculation unit, and the COP estimation calculation unit. The control unit switches the joint load calculation estimation unit 45, the muscle activity estimation calculation unit, the reaction force estimation calculation unit, and the COP estimation calculation unit according to the input information, for example, and outputs the input information to the switched functional unit to cause the functional unit to perform processing. One of the joint load calculation estimation unit 45, the muscle activity estimation calculation unit, the reaction force estimation calculation unit, and the COP estimation calculation unit may be replaced with the inside-outside body information calculation unit 19.

In this example, the joint load calculation estimation unit 45 is used, and the muscle activity estimation calculation unit, the reaction force estimation calculation unit, and the COP estimation calculation unit will be described later. The joint load calculation estimation unit 45 calculates (computes) a load (hereinafter referred to as joint load) on the joint as inside-outside body information on the basis of the joint position filter data from the low-pass filter 35 and the plurality of virtual markers from the virtual marker addition unit 39. Model data M2 is incorporated in the joint load calculation estimation unit 45, a weight assignment and a gravity center position of a segment (muscle and bone or bone and marker) at a definite height and weight are set in the model data, and upon input of a height 46 and a weight 47 of the evaluation subject and joint position filter data, scaling conversion of the model data is performed on the basis of them, and the model data is converted into model data (hereinafter referred to as evaluation subject model data) matching the skeleton of the evaluation subject. The joint load calculation estimation unit 45 performs inverse kinematics by using the evaluation subject model data obtained by the scaling conversion, the joint position filter data, the joint position filter data to which a virtual marker is added, and calculates a time-series joint angle of the evaluation subject model data. The joint load (joint torque) is obtained by calculation using inverse dynamics from a calculation result of the inverse kinematics, and is output to the second post-processing unit 21. As described above, the joint load calculation estimation unit 45 of the present example calculates and obtains the value of the joint load.

The height 46 and the weight 47 are measurement data at the time of evaluation of the evaluation subject. The height 46 and the weight 47 may be stored in advance in an evaluation-time data storage unit (not illustrated) at the time of measurement, or may be input to the joint load calculation estimation unit 45 by an input operation from the terminal device 12.

The second post-processing unit 21 is a low-pass filter, and this low-pass filter is configured similarly to the low-pass filter 35. In a case where time-series data of any one of the joint load, a muscle activity, a reaction force, and COP (time-series data of the joint load in this example) is input from the inside-outside body information calculation unit 19, the second post-processing unit 21 performs frequency filtering processing of removing high frequency components to obtain only low frequency components, and makes time-series data in which a temporal change of the joint load is smooth. The time-series data (hereinafter referred to as joint load filter data) of the joint load smoothly shifted by the second post-processing unit 21 is output to the data selection unit 41 and the data analysis unit 42 of the evaluation unit 25.

In the reference storage unit 23, at least one of standard data and history data that is various past data of the evaluation subject is stored as reference data. Various average values obtained from data of a plurality of persons sampled as evaluation reference persons and a numerical width including the average value (a range from (a first threshold smaller than the average value) to (a second threshold larger than the average value)) are set as reference information, and the standard data includes the plurality of pieces of reference information. The various average values are, for example, respective average values of height and weight, color-corrected moving image data, joint position filter data, joint angle time-series data, joint angular speed, joint position speed, joint load, joint load filter data, and the like. Note that the history data may be various past data of another person different from the evaluation subject.

The evaluation unit 25 includes the data selection unit 41 and the data analysis unit 42 described above. In a case where at least one of the joint position filter data as an output value from the posture estimation unit 15, the joint angle from the joint angle calculation unit 36, the joint angular speed and the joint position speed from the speed calculation unit 37, or the joint load filter data as an output value from the inside-outside body information calculation unit 19 is input, the data selection unit 41 selects a frame (referred to as a keyframe) to be evaluated among motions of the evaluation subject on the basis of the action type 49 checked in advance in an action type storage unit (not illustrated), and also selects a similar keyframe from the reference storage unit 23. A plurality of frames may be selected. The frame is numbered, and the data selection unit 41 outputs the number (keyframe number) of the keyframe selected as a feature point selection result to the data analysis unit 42 and the display control unit 27. Examples of the action type 49 include walking and batting swing. The action type storage unit may be provided outside the motion evaluation device 11, or may be provided in the motion evaluation device 11 as a functional unit constituting the motion evaluation device 11.

In a case of evaluating a case where the motion of the body is walking, for example, a frame image indicating the grounding of the heel on one side and a frame image of the next grounding of the heel on the same side are specified and selected as the first keyframe image and the second keyframe, respectively. Then, by generating a predetermined number of frame images by resampling all the inside-outside body information such as the joint angle and/or the joint torque from the first keyframe image to the second keyframe image, comparison with the reference data becomes easy. Similarly, for the reference data, it is preferable to specify and select the first keyframe image and the second keyframe image from the time-series data acquired in advance, resample the data for the first keyframe image to the second keyframe image, and obtain the predetermined number of frame images. Thus, since pieces of time-series data having the same number of frame images are compared with each other, processing efficiency is further improved, and a highly accurate evaluation result can be obtained.

In a case where the swing of batting in baseball is evaluated as a motion of the body, a plurality of keyframes can be defined, such as a time point when the front foot starts to take off from the stance posture, a time point when the knee is highest, a time point when the front foot touches the ground, a time point when the upper limb faces forward, and a time point when the turning of the upper body including the trunk and the upper limb is stopped and the swing ends. By selecting and resampling these keyframes, it is possible to compare the action in the reference storage unit 23 and the time series in synchronization. Note that the reference data may be data known in a paper or the like, or may be past data regarding the evaluation subject.

In a case where the frame of the keyframe number input from the data selection unit 41 is input, the data analysis unit 42 calculates the following 1 to 3.

    1. Difference between the reference data from the reference storage unit 23 and various data (color-corrected moving image data, joint position filter data, joint angle, joint angular speed, joint position speed, and joint load filter data) obtained for the evaluation subject, the difference being obtained for each type of data
    2. Evaluation value indicated by 0 to 5 of one decimal place for each of the various data based on the above 1
    3. 0 to 5 total evaluation values of one decimal place based on the evaluation values of 2 above

Specifically, it is as follows. The data analysis unit 42 calculates a loss value L for each of the joint position, the joint angle, the joint angular speed, the joint position speed, and the like using the following Expression (A) with respect to the frame indicated by the keyframe number. Symbols in Expression (A) mean the following.
    $i$; Number of each joint or inside-outside body information ($i$= an integer of 1 or more and $n$ or less, and $n$ is the obtained total number)
    $w_i$; Weight when the loss value L of the portion indicated by $i$ is calculated, and is set in advance for each type of action.

[Math. 1]

$$L = \frac{1}{n}\sum_{i=1}^{n}\left\{W_i \cdot \left(\left| \text{Various data}_i \text{ of evaluation subject - reference data}_i \right|\right)\right\} \quad \cdots \quad (A)$$

For the evaluation value F, a function of f(L) is prepared and set to a value of 0 to 5 of one decimal place, that is, 0.0 to 5.0. The total evaluation is the same as described above, and all of respective joints and respective pieces of inside-outside body information and the loss of all frames is biased by a certain weight and is set to a value of 0 to 5 of one decimal place by the function g.

As described above, the data analysis unit 42 obtains the above 1 to 3 on the basis of the joint position and the inside-outside body information obtained by the posture estimation unit 15 and the inside-outside body information calculation unit 19, that is, from the joint position filter data, the joint angle, and the joint position speed calculated on the basis of the joint position, and the joint load filter data calculated on the basis of the inside-outside body information. In this manner, the evaluation unit 25 evaluates a motion of the body of the evaluation subject on the basis of the reference information, the plurality of joint positions, and the inside-outside body information.

The display control unit 27 generates an image that displays at least one of the evaluation results of the above 1 to 3 and transmits the image to the display 29. The display control unit 27 may generate the image by further adding the data from the reference storage unit 23, the color-corrected image data, the keyframe number, or the time of the moving image corresponding to the keyframe number, or the keyframe indicated by the keyframe number, and this is also the case in the present example.

In the present example, the display 29 displays the color-corrected moving image data, the joint position filter data that is time-series data, and the like together with history data. Then, the motion evaluation device 11 is configured to display a radar chart, a graph having a vertical axis indicating a value of the display target and a horizontal axis that is a time axis, text display such as numerical values and characters, and a table by combining and switching the display and the table according to the display target. The display 29 displays the image acquired from the display control unit 27.

The motion evaluation device 11 is configured by a computer, and functions as each unit by executing a motion evaluation program. The motion evaluation program causes a computer to execute a reference storage step, a posture estimation step, an inside-outside body information calculation step, an evaluation step, and a display control step. The reference storage step stores reference information as a reference for evaluation. The posture estimation step estimates a joint position of each of a plurality of joints of an evaluation subject on the basis of moving image data of the evaluation subject evaluated for a motion of a body. The inside-outside body information calculation step obtains, as inside-outside body information, at least one of a muscle activity, a joint load, a center of pressure, or a reaction force for the evaluation subject by using a height and weight of the evaluation subject and model data. The evaluation step evaluates the motion of the body of the evaluation subject on the basis of the reference information, a plurality of the joint positions, and the inside-outside body information. The display control unit generates an image indicating an evaluation result obtained in the evaluation step.

In the image displayed on the display 29, data based on the joint load obtained by the inside-outside body information calculation unit 19 is displayed as an evaluation result. Therefore, the physical condition of the evaluation subject is more accurately grasped. Further, with the motion evaluation device 11, by including the posture estimation unit 15, the inside-outside body information calculation unit 19, and the like, the physical condition of the evaluation subject is quantified and more objectively obtained, and information regarding the physical condition that cannot be visually grasped is also revealed. Therefore, the evaluation result is obtained as quantitative information, and certain reliability is secured in the evaluation result. As a result, for example, in a case where advice (advice) is given on the basis of the obtained evaluation result, information in which common and certain reliability is ensured is shared among a plurality of staff members who perform rehabilitation of a patient, a plurality of trainers who instruct a person who performs sports, and the like, and for example, occurrence of a difference in recognition in mutual information handover is suppressed. Further, information can be shared remotely, and more detailed and accurate advice can be derived between staff members or trainers.

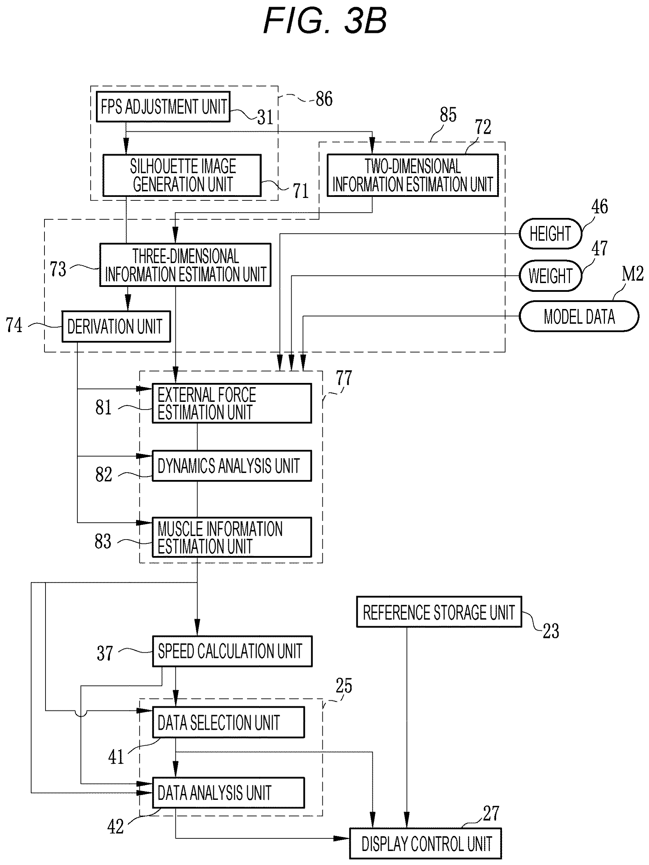

In the above example, each of positions of joints (hereinafter referred to as joint positions) of the evaluation subject is estimated using the posture estimation unit 15 in which the posture estimation model M1 and the AI are incorporated and output in three-dimensional coordinates, but is not limited thereto. For example, as illustrated in Fig. 3B, the color correction unit 32, the posture estimation unit 15, the first post-processing unit 17, and the inside-outside body information calculation unit 19 may be replaced with a silhouette image generation unit 71, a two-dimensional information estimation unit 72, a three-dimensional information estimation unit 73, a derivation unit 74, and an inside-outside body information calculation unit 77 described below. The inside-outside body information calculation unit 77 includes an external force estimation unit 81, a dynamics analysis unit 82, and a muscle information estimation unit 83. The two-dimensional information estimation unit 72, the three-dimensional information estimation unit 73, and the derivation unit 74 constitute a posture estimation unit 85. Note that, in this example, a pre-processing unit 86 including a silhouette image generation unit 71 instead of the color correction unit 32 described above is provided, and the speed calculation unit 37 of the first post-processing unit 17 is used.

In a case where the time-series data of frame images obtained through the resolution reduction processing and the FPS reduction processing is input, the silhouette image generation unit 71 generates a silhouette image of the evaluation subject who is the subject for each frame image in response to the input, and outputs the silhouette image to the three-dimensional information estimation unit. In the silhouette image, the inside of an outline indicating an outline of the evaluation subject is defined as the foreground, and classified as the background outside the outline. The silhouette image may be any of an image obtained by extracting the foreground, an image in which the foreground is depicted in a color different from the background, and an image in which the foreground is depicted in a pattern different from the background, and in this example, the silhouette image is an image obtained by extracting the foreground.

The two-dimensional information estimation unit 72 estimates, as two-dimensional information, time-series data (hereinafter referred to as two-dimensional time-series data) of joint positions (hereinafter referred to as two-dimensional joint positions) in two dimensions of the subject on the basis of time-series data of the frame images.

The two-dimensional joint position is a two-dimensional position of a plurality of joints such as an elbow, a wrist, a finger joint, a neck, and a knee, for example, in each frame image of the time-series data.

The two-dimensional joint positions are expressed in an image coordinate system. In the image coordinate system, a vertex located at the leftmost and uppermost corner of the image region defined by an xy plane is regarded as an origin (0, 0), a direction toward the right is regarded as a positive direction of the x axis (positive value gradually increases), and a direction toward the bottom is regarded as a negative direction of the y axis (negative value gradually decreases). The two-dimensional information estimation unit 72 outputs the two-dimensional information obtained by the estimation to the three-dimensional information estimation unit 73.

A method for estimating the two-dimensional information is not particularly limited, and a known method can be used. For example, as for the two-dimensional joint positions, time-series data of frame images constituting moving image data acquired in advance, such as DeepPose (Non Patent Literature 1: A. Toshev and C. Szegedy, "DeepPose: Human Pose Estimation via Deep Neural Networks," 2014 IEEE Conference on Computer Vision and Pattern Recognition, Columbus, OH, USA, 2014, pp. 1653-1660), is input to a convolutional neural network, and each joint point on the image coordinate system can be estimated by regression. The two-dimensional joint positions may be estimated using an improved method in which the probability distribution of the joint positions is expressed by a heat map or the estimation accuracy is improved by grouping of joint candidate points, like OpenPose (Non Patent Literature 2: Z. Cao, G. Hidalgo, T Simon, S. Wei, and Y. Sheikh. 2021. "OpenPose: Realtime Multi-Person 2D Pose Estimation Using Part Affinity Fields," IEEE Trans. Pattern Anal. Mach. Intell. 43, 1 (Jan. 2021), 172-186).

Since the two-dimensional information estimation unit 72 obtains the two-dimensional information from the moving image captured by only one camera 12a, only one terminal device 12 is installed under the evaluation subject P. Therefore, space saving of installation is achieved, and a device configuration and a system configuration are simple. Further, since it is only necessary to capture an image by the camera 12a of the terminal device 12 and acquire the motion of the evaluation subject P as a moving image, preparation for starting evaluation is also saved.

Upon input of time-series data of the silhouette image from the silhouette image generation unit 71 and the two-dimensional information from the two-dimensional information estimation unit 72, the three-dimensional information estimation unit 73 estimates motion information and the position information and the angle of the floor surface based on the two-dimensional information in response to the input. The motion information includes first data of a dimension of a position and second data of a dimension of a speed, which are smoothly displaced along a time series and expressed in a generalized coordinate system, for the evaluation subject P. For example, in a case where the time-series data of the two-dimensional joint position is received as the two-dimensional information, the three-dimensional information estimation unit 73 generates the first data of the dimension of the position indicated by the generalized coordinate system and the second data of the dimension of the speed while the two-dimensional joint position is smoothly displaced along the time series. The three-dimensional information estimation unit 73 estimates the three-dimensional position and angle (angle with respect to the horizontal plane) of the floor surface on the basis of the motion information. The first data and the second data are time-series data, the first data is $q_m$ to be described later, and the second data is $q_m'$ to be described later. The three-dimensional information estimation unit outputs the estimated first data and second data to the derivation unit 74, and outputs the three-dimensional position and angle of the floor surface to the external force estimation unit 81.

Here, the generalized coordinate system will be described. The generalized coordinate system is a coordinate system that can express the posture of the evaluation subject P by a variable according to the degree of freedom of the model in a case where the evaluation subject P is modeled. In the present embodiment, for example, similarly to the generalized coordinate system described in JP 2020-201138 A, the generalized coordinate system defines a joint JT(i) connecting segments with each of the head, the chest, the abdomen, the pelvis, the left and right thighs, the left and right shin portions, and the left and right legs as a segment SG (where i=1 to N). A segment SG(B) corresponding to the pelvis among segments SG is defined as a base segment SG(B). A six-degree-of-freedom displacement of the segment SG other than the base segment SG(B) with respect to an absolute coordinate system origin is not a variable of the generalized coordinate system, and only the base segment SG(B) is treated as being displaceable with six degrees of freedom. The six degrees of freedom include three directions of translation (XYZ) and three directions of rotation (yaw, roll, and pitch).

The posture (hereinafter referred to as a "generalized position") $q_m$ of the evaluation subject P expressed by the generalized coordinate system is a group of variables having a dimension of a position, and is expressed by, for example, the following Expression (1). Note that, in the following description, ' represents a first order differential, and " represents a second order differential. In Expression (1), $q_0$ is a vector of a total of seven elements including four elements of an X coordinate, a Y coordinate, a Z coordinate, and a quaternion representing the posture of the base segment SG(B). $q_i$ is a vector of the rotation angle of the number of dimensions according to the degree of freedom for each joint JT(i) (where i = 1 to N). For example, a joint corresponding to the knee is defined to have two degrees of freedom in a bending and stretching direction of the knee and a torsional direction of the shin, and thus is expressed by a two-dimensional vector. Further, since the joint corresponding to the hip joint is defined to have three degrees of freedom, the joint is expressed by a three-dimensional vector.

[Math. 2]

$$q_m = [q_0^T, \; q_1^T, \; \cdots q_i^T, \; \cdots q_N^T] \qquad \cdots (1)$$

The three-dimensional information estimation unit 73 is, for example, a machine learning model that generates data of a dimension of a position regarding the evaluation subject P expressed in a three-dimensional generalized coordinate system on the basis of the two-dimensional joint position input from the two-dimensional information estimation unit 72. The three-dimensional information estimation unit 73 may generate the generalized positions $q_m$ and $q_m'$ so as to coincide with the acquired two-dimensional joint positions using a data generation method such as a conditional variational autoencoder (CVAE). The CVAE exemplified here generates three-dimensional motion information of the evaluation subject P smoothly in time series and in conformity with the floor surface. Here, $q_m$ and $q_m'$, which are motion information, are set as a state variable x.

[Math. 3]

$$x = [ q_m, q_m' ] \qquad \cdots (2)$$

The CVAE is a machine learning model that generates new data on the basis of information of a certain state (condition). In this example, in order to generate xt, which is a state variable x at time t, $x_{t-1}$, which is the state variable x at time t-1, is set to condition. That is, in a case where $x_{t-1}$ is input, the CVAE generates the next state variable xt having the highest probability as the motion state. This probability can be obtained from a probability distribution obtained by learning a data set including time-series data (time-series motion data) of a plurality of motions. This probability distribution is a prior establishment distribution learned on a latent space of machine learning and is defined as a latent variable $z_t$. Therefore, the probability distribution in the learning parameter θ can be expressed by the Gaussian distribution formula of the following Expression (3) using the average value $\mu_\theta$ and the standard deviation $\sigma_\theta$. In Expression (3) and Expressions (11) and (12) described later, N (in the expression, italic characters (italic characters)) represents a Gaussian distribution.
[Math. 4]

$$\mathcal{N}( z_t ; \mu_\theta (x_{t-1}), \sigma_\theta (x_{t-1})) \qquad \cdots (3)$$

Furthermore, the relationship between xt and $z_t$ can be expressed as the following expression (4) using the change amount $\Delta\theta$ from $x_{t-1}$ to xt.
[Math. 5]

$$x_t = x_{t-1} + \Delta\theta (z_t, x_{t-1})) \qquad \cdots (4)$$

Therefore, since $\Delta\theta$ is derived from information of $x_{t-1}$, xt is generated. Since $x_0$, which is motion information at t = 0, cannot be derived from the above Expression (4), it is preferable to generate $x_0$ by creating a probability distribution of x at t = 0 using the Gaussian mixture model.

Many pairs of xt and $x_{t-1}$ are used as a data set (motion data set) of motion data for learning the CVAE. By way of example, motion data may be generated using human $q_m$ and $q_m'$ expressed in a generalized coordinate system calculated using inverse kinematics calculations on the basis of marker information acquired by optical motion capture. Alternatively, it may be acquired by a method using an inertial measurement unit (IMU, inertial sensor) or a video camera.

For learning of the CVAE, a learning parameter θ that minimizes the loss function L is searched. The loss function L can be expressed by the following Expression (5).
[Math. 6]

$$L = L_{rec} + L_{joint} + L_{contact} \qquad \cdots (5)$$

$L_{rec}$ is an L2 norm of xt that is teacher data and xt hat that is reconfigured on the basis of $x_{t-1}$, and is expressed by Expression (6).
[Math. 7]

$$L_{rec} = ||x_t - \hat{x}_t||^2 \qquad \cdots (6)$$

$L_{joint}$ is an L2 norm of a joint position Jt of a rigid link model obtained from xt of the teacher data and the joint position Jt hat of the rigid link model obtained from the reconstructed xt hat, and is expressed by Expression 7. Jt represents X, Y, and Z coordinate components of $q_{mt}$ which is $q_m$ at time t.
[Math. 8]

$$L_{joint} = ||J_t - \hat{J}_t||^2 \qquad \cdots (7)$$

$L_{contact}$ is a loss function that evaluates a ground state between a contact point in contact with the floor surface of the foot and the floor surface. Here, the CVAE can be reconfigured by estimating a variable $c_t$ hat indicating the probability that the contact point is grounded to the floor surface at the same time as xt. The teacher data ct[0,1] in the ground state, which is binary data, is obtained from the data of the position and speed of the contact point obtained from xt. It is preferable that a first threshold TH1 of the height of the contact point from the floor surface and a second threshold TH2 of the speed of the contact point are set in advance, and contact with the ground may be determined in a case where the height of the contact point from the floor surface is equal to or less than TH1 and the speed of the contact point is equal to or less than TH2. Although TH1 and TH2 are not particularly limited, in the present example, TH1 is set to, for example, 0.05 m, and the second threshold to 0.8 m/s, so as to determine the contact with the ground. $L_{contact}$ includes $L_{BCE}$ and $L_{vel}$, and can be expressed by Expression (8). The $L_{BCE}$ obtains the teacher data ct and the loss of the reconstructed $c_t$ hat using the binary cross entropy, and increases the estimation accuracy of the ground state by the CVAE. $L_{vel}$ is expressed by Expression (9) and serves to suppress an increase in speed vt hat of the contact point in a contact state.

[Math. 9]

$$L_{contact} = L_{BCE} + L_{vel} \qquad \cdots \ (8)$$

[Math. 10]

$$L_{vel} = \hat{c}_t \, ||\, \hat{v}_t \,||^2 \qquad \cdots \ (9)$$

A motion data set similar to the CVAE can be used for learning the Gaussian mixture model. Here, a method of estimating xt hat on the basis of the two-dimensional joint position acquired from the two-dimensional information estimation unit 72 will be described.

The learned CVAE is a deep learning model capable of generating three-dimensional motion information $x_{0:T}$ which is time-series data from the observed two-dimensional joint position to the last time T of observation. First, $x_0$ is generated from the Gaussian mixture model. In a case where $x_{t-1}$ is input to the CVAE, in response to this input, $z_t$ is sampled to generate xt. Thus, in a case where $x_0$ is input to the CVAE, $z_1$ is sampled in response to this input to generate $x_1$. By repeating this until time T, $z_{1:T}$, the latent variable z and the state variable x from t = 1 to t = T, can be sampled to generate $x_{1:T}$. At this generation time point, $x_{0:T}$ does not match the information of the observed two-dimensional joint position, and an error occurs. Accordingly, the final estimated motion information x hat is acquired by performing optimization calculation to obtain an optimum solution of $x_0$ and $z_{1:T}$ that minimizes this error. Furthermore, since the observed two-dimensional joint positions do not include information on the position of the floor surface, dimension data g of the position of the floor surface is similarly optimized. The entire objective function can be expressed by the following Expression (10).

[Math. 11]

$$\min_{x_0, z_{1:T}, g_1} \varepsilon_{mot} + \varepsilon_{data} + \varepsilon_{reg} \qquad \cdots \ (10)$$

[Math. 12]

$$\varepsilon_{CVAE} = -\lambda_{CVAE} \sum_{t=1}^{T} \log \mathcal{N}\left(z_t; \mu_\theta(x_{t-1}), \sigma_\theta(x_{t-1})\right) \qquad \cdots \ (11)$$

$\varepsilon_{mot}$ is expressed by Expression (11), and aims to maximize the log likelihood for the probability distribution of the CVAE and the Gaussian mixture model so that $\varepsilon_{CVAE}$ and $\varepsilon_{init}$ are expressed by the following Expression (12), and thus the closer to the center of the probability distribution, the lower the optimization cost. Hereinafter, $\lambda$ is a weight for each term.

[Math. 13]

$$\varepsilon_{init} = -\lambda_{init} \log \sum_{i=1}^{K} \gamma^i \mathcal{N}\left(x_0; \mu_\theta^i, \sigma_\theta^i\right) \qquad \cdots \ (12)$$

$\varepsilon_{data}$ in Expression (10) evaluates the observed data loss. $\varepsilon_{data}$ is expressed by the following expression in a case where the two-dimensional joint position is input to the three-dimensional information estimation unit 73.

[Math. 14]

$$\varepsilon_{data} \triangleq \varepsilon_{data}^{2D} = \lambda_{data} \sum_{t=0}^{T} \sum_{j=1}^{J} \sigma_t^j \rho\left(\Pi(p_t^j) - y_t^j\right) \qquad \cdots \ (13)$$

In Expression (13), when "j" is a joint position, $P_t^j$ is a three-dimensional position of the joint j, and $\Pi$ is a function that represents a pinhole projection and projects the joint j on a two-dimensional image coordinate system using the position and focal length information of the camera 12a (see Fig. 2) used for image capturing. $y_t^j$ is a two-dimensional joint position on the observed image coordinate system. Therefore, joint position information calculated from the motion information generated by the optimization calculation is two-dimensionally projected, and Expression (13) works to minimize an error with the two-dimensional joint position obtained by the two-dimensional information estimation unit 72. $\sigma_t{}^j$ is the reliability of the estimated two-dimensional joint position, and observation data with higher reliability is weighted more greatly. $\rho$ is a Geman-McClure function for improving robustness against outliers.

$\varepsilon_{reg}$ in Expression (10) is constituted by the following elements.
[Math. 15]

$$\varepsilon_{reg} = \varepsilon_{skel} + \varepsilon_{env} + \varepsilon_{gnd} \qquad \cdots \quad (14)$$

$\varepsilon_{skel}$ and $\varepsilon_{env}$ are terms set so that the motion information generated from the CVAE is smooth in time series and the consistency with the floor surface is improved.
[Math. 16]

$$\varepsilon_{skel} = \sum_{t=1}^{T} \left( \lambda_b \sum_{i=1}^{B} \left( l_t^i - l_{t-1}^i \right)^2 \right) \qquad \cdots \quad (15)$$

$l_t^i$ and $l_{t-1}^i$ are lengths of segments calculated from the generated motion information, and work to reduce a variation from time t to time t-1.
[Math. 17]

$$\varepsilon_{env} = \sum_{t=1}^{T} \sum_{j=1}^{J} \lambda_{CV} c_t^j || p_t^j - p_{t-1}^j ||^2 + \lambda_{ch} c_t^j \max \left( |p_{z,t}^j| - \delta, 0 \right) \qquad \cdots \quad (16)$$

$c_t$ is a ground contact probability of a contact point $p_t^j$ generated by the CVAE, and the first term of Expression (16) suppresses the movement when $p_t^j$ is in the ground state. The second term in Expression (16) works so that the height z of $p_t^j$ from the floor is lower than a threshold $\delta$ when the contact point $p_t^j$ is in the ground state.

0064 of P02A

$\varepsilon_{gnd}$ suppresses a change from an initial value $g_{init}$ in the optimization of position data of the floor surface by the following Formula (17). In this example, the initial value $g_{init}$ is a horizontal plane having a height of 0 m.
[Math. 18]

$$\varepsilon_{gnd} = \lambda_{gnd} || g - g_{init} ||^2 \qquad \cdots \quad (17)$$

By solving an optimization problem using the objective function described above, the three-dimensional time-series motion information $x_{0:T}$ represented by the generalized coordinate system is estimated.

Upon input of the second data from the three-dimensional information estimation unit 73, the derivation unit 74 first differentiates the second data in response to the input, thereby deriving third data of the dimension of acceleration expressed by the generalized coordinate system. The third data is time-series data. The derivation unit 74 of the present example first differentiates the generalized speed [q_m] ' to derive a generalized acceleration [q_m]" as the third data. The derivation unit 74 outputs the first data and the second data input from the three-dimensional information estimation unit 73 to each of the external force estimation unit 81 and the dynamics analysis unit 82 in association with the derived third data. The derivation unit 74 also outputs the first data and the second data input from the three-dimensional information estimation unit 73 to the muscle information estimation unit 83.

Model data and AI of the posture estimation model are incorporated in the three-dimensional information estimation unit 73. In a case where the time-series data of the silhouette image is input from the silhouette image generation unit 71 and the time-series data of the two-dimensional joint position is input from the two-dimensional information estimation unit 72, the three-dimensional information estimation unit 73 estimates a posture in three dimensions (hereinafter referred to as a three-dimensional posture) by AI using the model data in response to these inputs. In this example, AI that generates data by a conditional variational autoencoder (CVAE) capable of giving a condition to generated data is used as the AI. In this AI, for example, a three-dimensional posture at time t+1 is estimated from a three-dimensional posture at time t by learning time-series data of the three-dimensional posture calculated from a skeleton model and inverse kinematics from optical motion capture data using a data set including set information in which the three-dimensional posture at time t that is a certain time point on the time axis is input information and the three-dimensional posture at time t+1 is output information. At this time, the three-dimensional joint position is projected on a two-dimensional plane on the image coordinate system described above using the internal parameters obtained by the calibration processing unit described above, and the optimum three-dimensional posture in which an error with the two-dimensional joint position obtained by the two-dimensional information estimation unit 72 is minimized is estimated. Therefore, in this example, the above-described calibration processing unit is used. In a case where the two-dimensional joint position obtained by the two-dimensional information estimation unit 72 is located outside the contour line of the silhouette indicated in the silhouette image, the silhouette image can be used as a determination material for determining as erroneous estimation and ignoring the estimation data. However, a method of acquiring three-dimensional information data in a case where the data set is created is not limited.

The above-described model data has 6890 vertices (hereinafter referred to as a skin vertex) set on the skin (body surface) of the body, and three adjacent vertices among the vertices constitute a plurality of triangular polygons, thereby being expressed as body surface data. The vertices are identified by IDs (identification information) from 0001 to 6890, and some of the vertices are associated with joint positions. Thus, IDs are specified (identified), resulting in unique skin vertex coordinates in any posture, allowing joint angles to be recalculated using inverse kinematics. The three-dimensional posture includes at least coordinates of joint positions, a joint rotation vector, skin vertex coordinates, and a position and a normal vector of a floor surface, which are indicated in three dimensions, and a ground contact timing when coming into contact with the floor surface, and the three-dimensional information estimation unit 73 estimates these as the three-dimensional posture to obtain the above-described three-dimensional information. Skin vertex coordinates are 6890 vertex coordinates on the body surface as described above. The floor surface normal vector is an upward unit vector having a start point on the floor surface and perpendicular to the floor surface.

Upon input of the first data to the third data from the derivation unit 74 and the three-dimensional position and angle of the floor surface from the three-dimensional information estimation unit 73, the external force estimation unit 81 estimates the external force from the floor surface contacting the evaluation subject P (see Fig. 2) by the dynamic analysis on the basis of the first data to the third data, the three-dimensional position and angle of the floor surface, and the above-described evaluation subject model data in response to these inputs. That is, the motion information is input to the above-described evaluation subject model data, and it is determined whether or not each contact point is in a contact state with the floor surface on the basis of the speeds of the plurality of contact points set and arranged on the sole of the foot and the height from the floor surface. Specifically, in a case where the speed of the contact point is equal to or less than the second threshold TH2 and the height is equal to or less than the first threshold, it is determined that the contact state is established, and in other cases, it is determined that the contact state is not established. A floor reaction force is generated from the contact point determined to be in a contact state with the floor surface. Accordingly, the external force estimation unit 81 performs dynamic analysis on the basis of the motion information, and estimates the external force from the contact point by optimization calculation so that a motion equation of the entire body is established. In the present example, the external force as the time-series data is estimated on the basis of the generalized position $q_m$, the generalized speed $q_m'$, the generalized acceleration $q_m''$, the three-dimensional position and angle of the floor surface, and the evaluation subject model data generated as described above.

The objective function can be expressed by the following Expression (18), and inverse dynamics calculation is performed.

[Math. 19]

$$\min_{F} \; \sum_{i=1}^{2N_f} || F_i ||^2$$

$$\text{s.t.} \; M(q_m)q_m'' + C(q_m, q_m') + G(q_m) + E = 0 \; ;$$

$$\forall_i \in [\![ 1, 2N_f ]\!], \; F_i \leq F_i^{max} \qquad \cdots \; (18)$$

In Expression (18), $F_i$ is a floor reaction force (vector) at $N_f$ contact points i present in each foot. The constraint $M(q_m)$ is a mass characteristic vector, $C(q_m, q_m')$ is a centrifugal force vector and a Coriolis force vector, and $G(q_m)$ is a gravity vector. E is an external force vector and is a sum of $F_i$. This constraint is a condition that no dynamic error occurs between the pelvis, which is the root joint, and the global space. $F_i$ is equal to or less than the maximum value $F_i^{max}$. The maximum value $F_i^{max}$ can be set to any value, and is obtained, in this example, by multiplying the weight BW by a gravitational acceleration, and further multiplying this multiplied value by 0.4. The value by which the multiplied value obtained by multiplying the weight BW by the gravitational acceleration is further multiplied is not limited to 0.4 in this example, and is preferably within a range of 0.28 or more and 0.52 or less, and by setting the value within this range, the error variation is suppressed to be quite small. The value by which the multiplied value obtained by multiplying the weight BW by the gravitational acceleration is further multiplied is particularly preferably 0.4 as in the present example, and it has been confirmed by sensitivity analysis that a change in error is reliably suppressed to less than 1% even when the value is changed to upper and lower 30%.

Upon input of the external force from the external force estimation unit 81 and the first data to the third data from the derivation unit 74, the dynamics analysis unit 82 performs the dynamics analysis on the basis of the external force, the first data to the third data, and an evaluation subject musculoskeletal model in response to these inputs. Thus, the dynamics analysis unit 82 calculates a joint torque of each of the plurality of joints connecting the plurality of segments.

In the dynamics analysis unit 82 of this example, a generalized force vector $\tau$ is calculated by the following Expression (19) by the inverse dynamics calculation. The generalized force vector $\tau$ includes forces in a translational direction and a rotational direction expressed in a generalized coordinate system, and the forces in the rotational direction are joint torques.

[Math. 20]

$$M(q_m)q_m'' + C(q_m, q_m') + G(q_m) = \tau \qquad \cdots (19)$$

Upon input of the first data and the second data from the derivation unit 74 and the joint torque from the dynamics analysis unit 82, the muscle information estimation unit 83 estimates at least one of the muscle activity and the muscle tension on the basis of the first data and the second data, the evaluation subject model data, and each joint torque in response to these inputs. Both the muscle activity and the muscle tension are time-series data.

The joint torque is generated by the muscle tension of a plurality of corresponding muscle models, and the muscle information estimation unit 83 distributes the joint torque to the muscle tension of each muscle model by optimization calculation. The optimization calculation is performed so that the sum of squares or the sum of cubes of the total body muscle activity is minimized under the constraint of a Hill-type muscle model. Thus, the muscle activity and the muscle tension are output as being more optimal. The muscle tension is obtained by the Hill-type muscle model by increase or decrease of the muscle activity, that is, the magnitude of the muscle activity that increases or decreases in time series, depending on the length of the muscle and the contraction speed (speed of change in length of the muscle) during the exercise. The length and the contraction speed of the muscle are determined from the first data and the second data.

Here, in Expression (20), "j" is the degree of joint freedom. In the present example, the relationship between a generalized force vector $\tau_j$ in the degree of joint freedom j and the muscle tension $F_m$ is expressed by the following Expressions (20) and (21). $a_m$ is a muscle activity of a muscle m, $F_m^0$ is the maximum muscle tension of the muscle m, $l_m$ is the muscle length, $v_m$ is the contraction speed of the muscle m, and $r_{m,j}$ is a moment arm of the muscle m in the degree of joint freedom j.

[Math. 21]

$$\sum_{m=1}^{n} \left[ a_m f \left( F_m^0, l_m, v_m \right) \right] r_{m,j} = \tau \qquad \cdots (20)$$

[Math. 22]

$$F_m = \left[ a_m f \left( F_m^0, l_m, v_m \right) \right] \qquad \cdots (21)$$

[Math. 23]

$$\min J = \sum_{m=1}^{n_m} \left( \frac{F_m}{F_{max,\, m}} \right)^2 \qquad \cdots (22)$$

[Math. 24]

$$\min J = \sum_{m=1}^{n_m} \left( \frac{F_m}{F_{max,\, m}} \right)^3 \qquad \cdots (23)$$

[Math. 25]

$$\min \ J \ = \ \sum_{m=1}^{n} \left( a_m \right)^p \qquad \cdots \ (24)$$

The muscle information estimation unit 83 distributes the generalized force vector $\tau_j$ to the muscle tension by solving an optimization problem using objective functions such as the above Expressions (22) to (24). J denotes an objective function and minJ denotes minimization of J. p is any index and is set in advance. p is preferably set within a range of 2 or more and 4 or less. In a case where the ratio is 2 or more, the estimation accuracy of the muscle activity is more reliably improved as compared with a case where the ratio is less than 2, and in a case where the ratio is 4 or less, the estimation accuracy of the muscle activity is more reliably improved as compared with a case where the ratio exceeds 4.

The inside-outside body information calculation units 19 and 77 can re-output the joint angle, and either the re-output joint angle or the joint angle output from the three-dimensional information estimation unit 73 may be used.

In the present example, at least one of the muscle activity or the muscle tension estimated as described above is transmitted to the terminal device 12 or the like and displayed on the display 29 which is the display unit of the terminal device 12 as described above. As described above, according to the present example, the joint torque and the muscle tension can be estimated even though only one camera 12a is used. Further, according to the above configuration, the three-dimensional position such as the joint position is accurately generated (estimated) even though only one camera 12a is used. Furthermore, since the external force from the floor surface is estimated after the three-dimensional position and angle of the floor surface are estimated, the joint torque and the muscle tension are estimated with high accuracy. Moreover, with the above configuration, since the three-dimensional positional relationship between the floor surface and the leg portion is also estimated, joint torque, muscle tension, muscle activity, and the like are required with quite high accuracy.

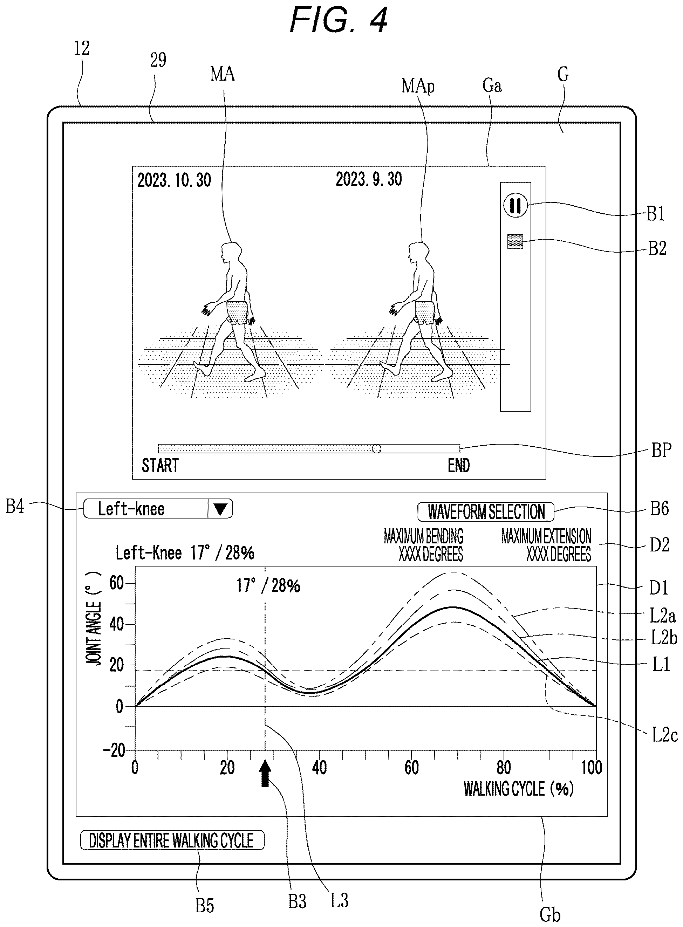

Upon input of data of an image to be displayed from the display control unit 27, the terminal device 12 displays the image G on the display 29 as illustrated in Fig. 4. The image G includes a moving image portion Ga indicating a moving image and a result image portion Gb indicating an evaluation result such as a graph and/or a numerical value. However, the moving image portion Ga and the result image portion Gb may form different images G to perform switching display.

In the moving image portion Ga, a moving image including a three-dimensional human body model MA illustrated as the evaluation subject P (see Fig. 2) is displayed, and the motion of the three-dimensional human body model MA in the moving image reflects the motion of the body of the evaluation subject P. In the example illustrated in Fig. 4, a moving image indicated by a three-dimensional human body model MA walking from the right side to the left side in Fig. 4 is displayed as the evaluation subject P walking on a flat floor surface. The moving image indicates at least from the start of walking to the end of walking, and the progress of the moving image is indicated by a progress status bar (progress bar) BP extending linearly from "START" indicating the start to "END" indicating the end. The image G includes a pause button B1 for pausing the moving image in progress, a stop button B2 for stopping the moving image in progress, a progress button (not illustrated) for progressing the moving image, and the like, and the progress of the moving image is turned on and off in response to a touch operation on these buttons.

In this example, since the three-dimensional human body model MA is generated by estimation of the three-dimensional information, the direction of the three-dimensional human body model MA can be changed by a swipe operation of moving a finger while contacting the moving image portion Ga of the display 29. Further, the grounding timing and the grounding position are also specified in a three-dimensional space, and the time-series data of them is obtained in association with each piece of time-series data of the joint position and the joint angle of the whole body, and thus, regardless of the direction of the three-dimensional human body model MA, the walking direction can be displayed as a moving image, and the sole is displayed in a state of being aligned with the floor surface. As described above, according to the motion evaluation system 10 (see Fig. 1), in addition to the system configuration being simple and space saving being achieved, the motion of the body of the evaluation subject P is reflected and displayed on the terminal device 12 by the three-dimensional human body model MA, and the display is variable in three dimensions and is aligned with the floor surface. As a result, the operator such as the evaluation subject P or the trainer using the terminal device 12 can repeatedly visually confirm the motion of the body of the evaluation subject P and can further consider the motion of the body. Further, as described above, the evaluation result for one walking cycle as described above from the first frame image to the second frame image is obtained from the time-series data from the walking start time to the walking end time, and resampling processing is performed on this walking cycle, and the reference information is also configured by the same number of frame images by the resampling processing. Therefore, the evaluation result can be illustrated in the result image portion Gb, and the current result of the evaluation subject P and the reference information are displayed with the same range in the same graph, and the processing time required for generating the image is also very small. As a result, although the system configuration is simple (simple), a detailed evaluation result is quickly displayed on the terminal device 12, and it is easy to grasp the current evaluation result of the evaluation subject P and to consider comparing the evaluation result with the reference information.

The moving image portion Ga may display a moving image including a three-dimensional human body model MAp together with a moving image including the three-dimensional human body model MA. The motion of the three-dimensional human body model MAp reflects the motion of the body of the evaluation subject P at the time of past evaluation "2023.9.30". On the other hand, at the time of the current evaluation, "2023.10.23" is illustrated in Fig. 4. The three-dimensional human body model MA and the three-dimensional human body model MAp may be displayed by enlarging or reducing one of the times so as to match their grounding timings with each other, or may be displayed by matching the time. In this example, since the grounding timing and the grounding position are specified in a three-dimensional space, any display is possible. In the example illustrated in Fig. 4, the three-dimensional human body model MA and the three-dimensional human body model MAp are displayed side by side in the lateral direction, but a positional relationship between the both may be another form such as side by side in the longitudinal direction. Further, in the example illustrated in Fig. 4, the three-dimensional human body model MA and the three-dimensional human body model MAp are displayed in substantially the same size, but either one may be displayed smaller than the other. Furthermore, an operation button (not illustrated) for switching on and off of display of the three-dimensional human body model MAp may be provided, and display and non-display of the three-dimensional human body model MAp may be switched by, for example, a touch operation on the operation button.

In the example illustrated in Fig. 4, the result image portion Gb includes, for "Left-knee" indicating the left knee joint, a graph D1 of the joint angle of the left knee joint in the one walking cycle and a numerical value display D2 indicating "maximum bending" that is a minimum value and "maximum extension" that is a maximum value of the joint angle. In the graph D1, a curve L1 of the joint angle of the left knee joint for the evaluation subject P and a curve (in Fig. 4, indicated by a two-dot chain line) L2a of the joint angle of the left knee joint as a reference walking variable are displayed in an overlapping manner. The reference walking variable is an example of the above-described standard data among the above-described reference data. In the example illustrated in Fig. 4, a curve L2b (in Fig. 4, indicated by a one-dot chain line) of the joint angle of the left knee joint as the history data and the joint angle of the right knee joint on the opposite side are displayed to be superimposed on the curve L1. Thus, it is easy to consider the evaluation subject P by comparing the past with the present. Note that the curve L2b indicating the history data is generated and displayed in conjunction with the time of evaluation "2023.9.30" of the three-dimensional human body model MAp indicated in the moving image portion Ga, but is not limited thereto. A curve L2c (Fig. 4 is indicated by a broken line) of the joint angle of the right knee joint on the opposite side may also be displayed to be superimposed on the curve L1. In this example, all of these curves L1 to L4 are displayed in an overlapping manner, whereby the motion of the body of the evaluation subject can be considered from various viewpoints. In the vicinity of the horizontal axis of the graph D1, there is provided a movement operation portion B3 indicated by an arrow, for example, for sliding a straight line L3 extending in the vertical direction, for example, indicated by a chain line, in a direction along the horizontal axis. By the client moving the movement operation portion B3 on the display 11a to a predetermined position on the horizontal axis in a state where the movement operation portion B3 is touched by the finger, the straight line L3 is displaced to the position, and the joint angle of the left knee joint and the walking cycle corresponding to the joint angle indicated by the curve L1 intersecting at the displaced position are displayed. For example, in Fig. 4, the joint angle and the walking cycle corresponding thereto are displayed as "17°/28%".

The result image portion Gb is provided with a switching operation unit B4 for switching the joint to be displayed in a pull-down manner. By the operator operating the terminal device 12 performing an operation of selecting another joint different from the left knee joint using the switching operation unit B4, the result image portion Gb including the graph D1 and the numerical value display D2 for the selected joint is displayed on the terminal device 12. Further, the result image portion Gb of this example is provided with a "waveform selection" button B6, so that a graph illustrating other evaluation results for the left knee joint is displayed.

The image G is further provided with a switch button B5 for displaying the evaluation result of the entire walking cycle, that is, the entire captured time. By the operator touching the switch button B5, the result image portion Gb is switched to a result image portion (not illustrated) indicating the evaluation result of the entire walking cycle. In this manner, the evaluation result of the entire walking cycle may be displayed similarly to the evaluation result of one walking cycle.

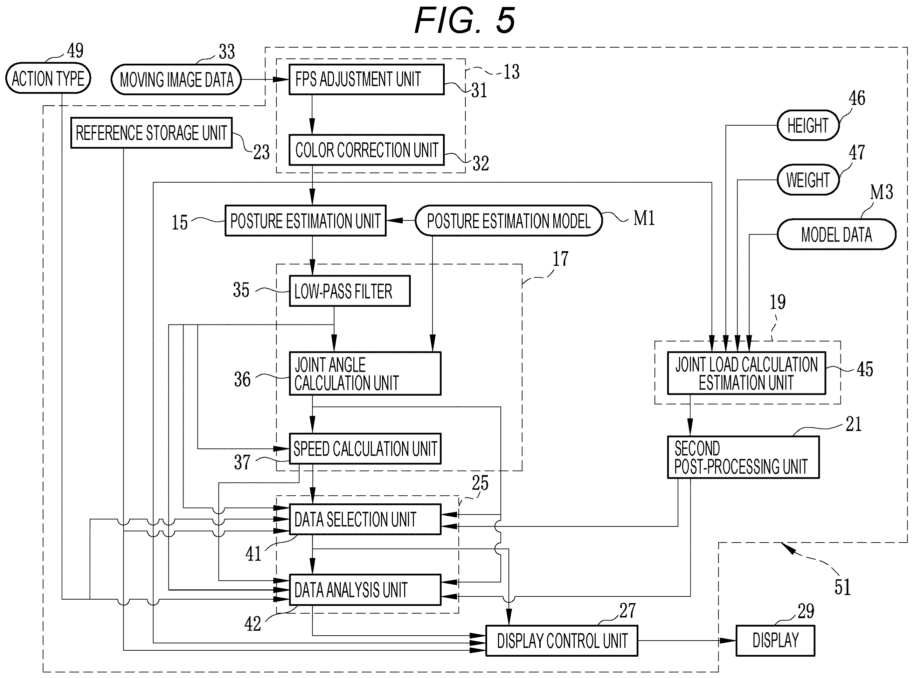

The motion evaluation device 51 illustrated in Fig. 5 is different from the motion evaluation device 11 (see Fig. 1) in that the virtual marker addition unit 39 is not provided, in that the color-corrected moving image data obtained by the color correction unit 32 is sent to the inside-outside body information calculation unit 19, in processing performed by the inside-outside body information calculation unit 19, and the like. Regarding the motion evaluation device 51, description of the same functional units and processing as those of the motion evaluation device 11 will be omitted.

The color correction unit 32 transmits the color-corrected moving image data to the joint load calculation estimation unit 45 of the inside-outside body calculation unit 19. The joint load calculation estimation unit 45 estimates a load (hereinafter referred to as joint load) on the joint as inside-outside body information on the basis of the color-corrected moving image data. The joint load calculation estimation unit 45 incorporates model data M3 different from the model data M2 and AI, and outputs the estimated value of the joint load to the second post-processing unit 21. As described above, the joint load calculation estimation unit 45 of the present example estimates and obtains the value of the joint load. As described above, the joint load calculation estimation unit 45 performs arithmetic processing of calculating or estimating the value of the joint load.

Also in the motion evaluation device 51, data based on the joint load obtained by the inside-outside body information calculation unit 19 is displayed as an evaluation result in the image displayed on the display 29. Therefore, the physical condition of the evaluation subject can be grasped more accurately, and the above-described effects obtained by the motion evaluation device 11 can be similarly obtained.

The inside-outside body information calculation unit 19 in each of the above examples only needs to output at least one or more of muscle activity, joint load, COP, or reaction force. The inside-outside body information calculation unit 19 may perform calculation for estimating or calculating the joint load, COP, and reaction force, and use calculation results at the time of calculation for estimating or calculating the muscle activity. Similarly, other output values may be used in a case where the joint load, COP, and reaction force are estimated or calculated. Furthermore, in a case where the joint position is used in the estimation calculation of the inside-outside body information calculation unit 19, it is different from a case of calculation operation in that AI is used.

The muscle activity estimation calculation unit, the reaction force estimation calculation unit, and the COP estimation calculation unit described above similarly perform either calculation processing for calculating a predetermined value or calculation processing for estimating the predetermined value. In a case of performing calculation operation, the muscle activity estimation calculation unit calculates and outputs the muscle activity of each muscle using the plurality of virtual markers added by the virtual marker addition unit 39, the joint position filter data, the height 46 and the weight 47, and the model data M2. The muscle activity of each muscle is calculated by performing optimization calculation (static optimization) on the basis of the joint load after the same calculation as the calculation performed by the joint load calculation estimation unit 45 illustrated in Fig. 3A.

The muscle activity estimation calculation unit incorporates AI in a case of performing estimation calculation, estimates muscle activity from moving image data in a case where the moving image data is input, and estimates muscle activity from joint position filter data in a case where the joint position filter data is input.

In a case of performing calculation operation, the reaction force estimation calculation unit calculates and outputs a reaction force (floor reaction force) using the plurality of virtual markers added by the virtual marker addition unit 39, the joint position filter data, the height 46 and the weight 47, and the model data M2. The calculation is performed as follows. That is, first, a plurality of contacts is assigned to a contact surface of an object (in this example, an evaluation subject) in contact with the ground (including the floor), and an actuator is arranged at each contact. Thereafter, in a case where the distance between the object and the ground becomes equal to or less than a certain value, the force on each contact point is calculated using the above-described calculation method performed by the joint load calculation estimation unit 45 in the motion evaluation device 11. The reaction force estimation calculation unit incorporates AI in a case of estimation calculation, estimates a reaction force from the moving image data in a case where the moving image data is input, and estimates the reaction force from the data in a case where the joint position filter data is input.

In a case of performing calculation operation, the COP estimation calculation unit calculates and outputs the COP by using the joint position filter data of the plurality of virtual markers added by the virtual marker addition unit 39, the height 46 and the weight 47, and the model data M2. The COP is obtained by using the reaction force of each contact point, the moment calculated from the reaction force, and the world coordinates of the contact surface. The reaction force estimation calculation unit incorporates AI in a case of estimation calculation, estimates a reaction force from the moving image data in a case where the moving image data is input, and estimates the COP from the data in a case where the joint position filter data is input.

In a case of performing the estimation calculation using the joint position filter data in the inside-outside body information calculation unit, two-dimensional joint position filter data may be used. Furthermore, the posture estimation unit 15 may output two-dimensional coordinates.

Reference Signs List

| | |
|---|---|
| 11, 51 | Motion evaluation device |
| 13, 86 | Pre-processing unit |
| 15, 85 | Posture estimation unit |
| 17 | First post-processing unit |
| 19, 77 | Inside-outside body information calculation unit |
| 21 | Second post-processing unit |
| 23 | Reference storage unit |
| 25 | Evaluation unit |
| 27 | Display control unit |
| 29 | Display |
| 31 | FPS adjustment unit |

**EP 4 699 531 A1**

32 Color correction unit
33 Moving image data
35 Low-pass filter
36 Joint angle calculation unit
37 Speed calculation unit
39 Virtual marker addition unit
41 Data selection unit
42 Data analysis unit
45 Joint load calculation estimation unit
46 Height
47 Weight
49 Action type
M1 Posture estimation model
M2, M3 Model data

**Claims**

1. A motion evaluation device (11) comprising:

a reference storage unit (23) configured to store reference information as a reference for evaluation;
a posture estimation unit (15) configured to estimate a joint position of each of a plurality of joints of an evaluation subject on a basis of moving image data of the evaluation subject evaluated for a motion of a body;
an inside-outside body information calculation unit (19) configured to obtain, as inside-outside body information, at least one of a muscle activity, a joint load, a center of pressure, or a reaction force for the evaluation subject by using a height and weight of the evaluation subject and model data;
an evaluation unit (25) configured to evaluate the motion of the body of the evaluation subject on a basis of the reference information, a plurality of the joint positions, and the inside-outside body information; and
a display control unit (27) configured to generate an image indicating an evaluation result obtained by the evaluation unit (25).

2. The motion evaluation device (11) according to claim 1, wherein the inside-outside body information calculation unit (19) obtains evaluation subject model data adapted to the evaluation subject by performing scaling conversion of the model data in which weight assignment and a gravity center position of a segment at a definite height and weight are set on a basis of the height and weight of the evaluation subject, and obtains the inside-outside body information using the obtained evaluation subject model data.

3. The motion evaluation device (11) according to claim 1 or 2, wherein the moving image data is data of a moving image captured from a fixed point with the evaluation subject as a subject.

4. The motion evaluation device (11) according to claim 1 or 2, wherein the evaluation unit (25) resamples the inside-outside body information from a first keyframe image to a second keyframe image selected from time-series data constituting the moving image data, and evaluates the motion of the body of the evaluation subject by comparing the inside-outside body information with the reference information.

5. A motion evaluation method comprising:

a reference storage step of storing reference information as a reference for evaluation;
a posture estimation step of estimating a joint position of each of a plurality of joints of an evaluation subject on a basis of moving image data of the evaluation subject evaluated for a motion of a body;
an inside-outside body information calculation step of obtaining, as inside-outside body information, at least one of a muscle activity, a joint load, a center of pressure, or a reaction force for the evaluation subject by using a height and weight of the evaluation subject and model data;
an evaluation step of evaluating the motion of the body of the evaluation subject on a basis of the reference information, a plurality of the joint positions, and the inside-outside body information; and
a display control step of generating an image indicating an evaluation result obtained in the evaluation step.

6. A motion evaluation program for causing a computer to execute:

a reference storage step of storing reference information as a reference for evaluation;

a posture estimation step of estimating a joint position of each of a plurality of joints of an evaluation subject on a basis of moving image data of the evaluation subject evaluated for a motion of a body;

an inside-outside body information calculation step of obtaining, as inside-outside body information, at least one of a muscle activity, a joint load, a center of pressure, or a reaction force for the evaluation subject by using a height and weight of the evaluation subject and model data;

an evaluation step of evaluating the motion of the body of the evaluation subject on a basis of the reference information, a plurality of the joint positions, and the inside-outside body information; and

a display control step of generating an image indicating an evaluation result obtained in the evaluation step.

# FIG. 1

# FIG. 2

FIG. 3A

# FIG. 3B

# FIG. 4

2023. 10. 30          2023. 9. 30

START          END

Left-knee ▼          WAVEFORM SELECTION

Left-Knee 17° / 28%          MAXIMUM BENDING          MAXIMUM EXTENSION
                              XXXX DEGREES              XXXX DEGREES

17° / 28%

JOINT ANGLE (°)

60

40

20

0

-20

0          20          40          60          80          100

WALKING CYCLE (%)

DISPLAY ENTIRE WALKING CYCLE

## FIG. 5

ACTION TYPE ~49

MOVING IMAGE DATA ~33 → FPS ADJUSTMENT UNIT ~31 ~13

REFERENCE STORAGE UNIT ~23

COLOR CORRECTION UNIT ~32

HEIGHT ~46

WEIGHT ~47

M3

POSTURE ESTIMATION UNIT ~15 ← POSTURE ESTIMATION MODEL ~M1

MODEL DATA

LOW-PASS FILTER ~35

17

JOINT ANGLE CALCULATION UNIT ~36

19

JOINT LOAD CALCULATION ESTIMATION UNIT ~45

SPEED CALCULATION UNIT ~37

25

SECOND POST-PROCESSING UNIT ~21

DATA SELECTION UNIT ~41

DATA ANALYSIS UNIT ~42

51

DISPLAY CONTROL UNIT ~27 → DISPLAY ~29

EP 4 699 531 A1

24

## INTERNATIONAL SEARCH REPORT

| | International application No. |
| --- | --- |
| | **PCT/JP2024/015298** |

**A. CLASSIFICATION OF SUBJECT MATTER**

*A61B 5/11*(2006.01)i; *A61B 5/107*(2006.01)i
FI:  A61B5/11 230; A61B5/107 300; A61B5/11 120

According to International Patent Classification (IPC) or to both national classification and IPC

**B. FIELDS SEARCHED**

Minimum documentation searched (classification system followed by classification symbols)

A61B5/11; A61B5/107; A63B69/00; A63B71/06; G06T7/00 ; G06T7/20

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2021-49208 A (SPORTIP CO., LTD.) 01 April 2021 (2021-04-01)<br>paragraphs [0012]-[0074], fig. 1-16 | 1-6 |
| A | JP 2022-51173 A (HITACHI, LTD.) 31 March 2022 (2022-03-31)<br>paragraphs [0013]-[0096], fig. 1-20 | 1-6 |
| A | JP 2020-141806 A (SPORTIP CO., LTD.) 10 September 2020 (2020-09-10)<br>paragraphs [0012]-[0075], fig. 1-20 | 1-6 |
| A | JP 2020-86823 A (FUJITSU ADVANCED ENGINEERING LTD.) 04 June 2020<br>(2020-06-04)<br>paragraphs [0013]-[0066], fig. 1-17 | 1-6 |
| A | JP 2021-83562 A (TOKYO METROPOLITAN PUBLIC UNIV. CORP.) 03 June 2021<br>(2021-06-03)<br>paragraphs [0016]-[0076], fig. 1-10 | 1-6 |

☐ Further documents are listed in the continuation of Box C.    ☑ See patent family annex.

| | |
| --- | --- |
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **26 June 2024** | **09 July 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

International application No.

**PCT/JP2024/015298**

| Patent document cited in search report | | | Publication date (day/month/year) | Patent family member(s) | Publication date (day/month/year) |
|---|---|---|---|---|---|
| JP | 2021-49208 | A | 01 April 2021 | (Family: none) | |
| JP | 2022-51173 | A | 31 March 2022 | WO 2022/059228 A1 paragraphs [0013]-[0096], fig. 1-20 | |
| JP | 2020-141806 | A | 10 September 2020 | JP 2022-43264 A paragraphs [0012]-[0075], fig. 1-20 | |
| JP | 2020-86823 | A | 04 June 2020 | (Family: none) | |
| JP | 2021-83562 | A | 03 June 2021 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022043264 A

- JP 2020201138 A

**Non-patent literature cited in the description**

- **A. TOSHEV** ; **C. SZEGEDY**. DeepPose: Human Pose Estimation via Deep Neural Networks. *2014 IEEE Conference on Computer Vision and Pattern Recognition*, 2014, 1653-1660

- **Z. CAO** ; **G. HIDALGO** ; **T SIMON** ; **S. WEI** ; **Y. SHEIKH**. OpenPose: Realtime Multi-Person 2D Pose Estimation Using Part Affinity Fields. IEEE Trans. Pattern Anal. Mach. Intell., January 2021, vol. 43, 172-186